# EUROPEAN PATENT APPLICATION

(11) **EP 4 609 858 A1**
(43) Date of publication of application: **03.09.2025**
(21) Application number: 24796110.5
(22) Date of filing: 24.04.2024
(51) Int. Cl.: A61K 9/16, A61K 31/485

(54) **BUPRENORPHINE IMPLANT AND PREPARATION METHOD THEREFOR**

(30) Priority: 25.04.2023 CN 202310472238
(71) Applicant: Shenzhen Sciencare Pharmaceutical Co., Ltd., Shenzhen, Guangdong 518118 (CN)
(72) Inventor: LIU, Daichun, Shenzhen, Guangdong 518118 (CN); QU, Wei, Shenzhen, Guangdong 518118 (CN); YAN, Xieguo, Shenzhen, Guangdong 518118 (CN); YIN, Shugui, Shenzhen, Guangdong 518118 (CN); ZHANG, Tao, Shenzhen, Guangdong 518118 (CN); CHEN, Zeqin, Shenzhen, Guangdong 518118 (CN); FU, Xiaofang, Shenzhen, Guangdong 518118 (CN); LUO, Jingling, Shenzhen, Guangdong 518118 (CN); LIANG, Shuhui, Shenzhen, Guangdong 518118 (CN); HU, Lijun, Shenzhen, Guangdong 518118 (CN); QIU, Xinmin, Shenzhen, Guangdong 518118 (CN)
(74) Representative: karo IP
(86) International application number: PCT/CN2024/089526
(87) International publication number: WO 2024/222733

(57) **Abstract**

A buprenorphine implant is provided, including: a biodegradable polymer and an active pharmaceutical ingredient dispersed in the biodegradable polymer. The active pharmaceutical ingredient includes one or more of buprenorphine and a pharmaceutically acceptable salt thereof. A plurality of pores are formed in an interior of the buprenorphine implant, with a porosity of 2% to 10% and an average pore size of 2 to 15 µm. When the porosity, average pore size, and pore distribution uniformity of the buprenorphine implant fall within the above ranges, the implant releases the drug continuously and steadily, thereby reducing the occurrence of drug burst release and delayed release from the implant.

## Description

### Cross-reference to Related Applications

This application claims the priority of Chinese patent application No. 202310472238.3, entitled "Buprenorphine Implant and Preparation Method Thereof" and filed on April 25, 2023, the entirety of which is incorporated herein by reference.

### Field of the Invention

The present application relates to the technical field of implants, and in particular, to a buprenorphine implant and a preparation method thereof.

### Background of the Invention

Buprenorphine, also known as "Shudingfei", is commonly used in its hydrochloride salt form (buprenorphine hydrochloride), with a molecular formula of C₂₉H₄₁NO₄·HCL and a molecular weight of 504.1. It is a white crystalline powder, slightly soluble in water, soluble in alcohol, and freely soluble in methanol. Buprenorphine exhibits antagonistic activity at δ and κ opioid receptors, avoiding psychotomimetic effects and dysphoric reactions such as agitation associated with some mixed opioid receptor agonist-antagonists such as cyclazocine. Buprenorphine was synthesized in the late 1960s, and its injectable form was initially used for pain relief. It was later adopted for detoxification treatment and as a substitution therapy for opioid dependence. Due to its potent analgesic effects, effective substitution therapy, and lower dependency potential, buprenorphine is considered safer and more reliable than morphine and methadone, leading to its widespread use.

A marketed buprenorphine implant, Probuphine, is prepared by mixing buprenorphine hydrochloride and ethylene-vinyl acetate (EVA) copolymer and then performing hot-melt extrusion. The drug is released rapidly in the initial phase of administration, followed by a gradual decline in plasma concentration, which eventually stabilizes over time. This technology faces two main problems: first, EVA is non-biodegradable in the body, requiring surgical removal after the treatment period, which negatively impacts patient compliance; second, there is a risk of burst release in the initial phase, followed by slow release in the middle and later phases, which may not maintain an effective plasma concentration.

### Summary of the Invention

Based on this, it is necessary to provide a biodegradable buprenorphine implant capable of reducing burst release or delayed release and a preparation method thereof.

In a first aspect of the present application, a buprenorphine implant is provided and includes:
a biodegradable polymer, and
an active pharmaceutical ingredient, dispersed in the biodegradable polymer and including one or more of buprenorphine and a pharmaceutically acceptable salt thereof,
a plurality of pores being formed in an interior of the buprenorphine implant, with a porosity of 2% to 10% and an average pore size of 2 to 15 µm.

In some embodiments, the active pharmaceutical ingredient accounts for 30% to 70% of the total mass of the active pharmaceutical ingredient and the biodegradable polymer, optionally 35% to 65%, and further optionally 40% to 60%.

In some embodiments, a particle size of the active pharmaceutical ingredient is ≤ 30 mesh,
optionally, the particle size of the active pharmaceutical ingredient is ≤40 mesh, and
further optionally, the particle size of the active pharmaceutical ingredient is ≤50 mesh.

In some embodiments, the biodegradable polymer has at least one of the following features:
(1) a glass transition temperature of the biodegradable polymer being 35°C to 65°C; and
(2) an end-capping group of the biodegradable polymer being an alkyl ester group or a carboxyl group.

In some embodiments, the biodegradable polymer further has at least one of the following features:
(3) a weight-average molecular weight of the biodegradable polymer being 5,000 to 100,000 Da and optionally 15,000 to 70,000 Da; and
(4) an intrinsic viscosity of the biodegradable polymer being 0.08 to 0.6 dl/g and optionally 0.15 to 0.5 dl/g.

In some embodiments, the biodegradable polymer includes one or more of polylactide, poly(lactide-co-glycolide), polylactide and polyethylene glycol copolymers, and poly(lactide-co-glycolide) and polyethylene glycol copolymers;
optionally, the biodegradable polymer includes one or more of polylactide and poly(lactide-co-glycolide); and
further optionally, the biodegradable polymer includes poly(lactide-co-glycolide).

In some embodiments, poly(lactide-co-glycolide) includes a lactide unit and a glycolide unit, and the molar percentage of the lactide unit in the total molar amount of the lactide unit and the glycolide unit is denoted as R, where 50% ≤ R < 100%, and optionally 65% ≤ R < 100%.

In some embodiments, the pharmaceutically acceptable salt of buprenorphine includes one or more of buprenorphine hydrochloride, buprenorphine salicylate, buprenorphine sulfate, buprenorphine fumarate, buprenorphine oxalate, buprenorphine hydrobromide, buprenorphine tartrate, buprenorphine maleate, and buprenorphine phosphate; and
optionally, the pharmaceutically acceptable salt of buprenorphine includes buprenorphine hydrochloride.

In some embodiments, the buprenorphine implant is cylindrical or rod-shaped; and
optionally, a diameter of the buprenorphine implant is ≤3 mm.

In some embodiments, the pores are not interconnected.

In a second aspect of the present application, a preparation method of the buprenorphine implant according to the first aspect is provided and includes the following steps:
preparing a mixture of an active pharmaceutical ingredient and a biodegradable polymer; and
performing hot-melt extrusion on the mixture to prepare the buprenorphine implant.

In some embodiments, a particle size of the active pharmaceutical ingredient and a particle size of the biodegradable polymer in the mixture are independently ≤30 mesh;
optionally, the particle size of the active pharmaceutical ingredient and the particle size of the biodegradable polymer in the mixture are independently ≤40 mesh; and
further optionally, the particle size of the active pharmaceutical ingredient and the particle size of the biodegradable polymer in the mixture are independently ≤50 mesh.

In some embodiments, moisture content of the active pharmaceutical ingredient and moisture content of the biodegradable polymer in the mixture are independently 1.5 wt% to 4.5 wt%.

In some embodiments, the hot-melt extrusion has at least one of the following features:
(1) a melt temperature of 95°C to 120°C;
(2) a twin-screw torque of 35% to 80%; and
(3) a die pressure of 30 Bar to 70 Bar.

In some embodiments, a parameter setting during hot-melt extrusion includes:
a twin-screw rotation speed of 50 rpm to 300 rpm; and optionally 80 rpm to 250 rpm.

According to the buprenorphine implant and the preparation method thereof, when the porosity of the buprenorphine implant reaches 2% to 10% and the average pore size is 2 µm to 15 µm, a suitable number of pathways are provided for the biodegradation or erosion of the biodegradable polymer by a subcutaneous tissue fluid or enzyme-catalyzed reactions. During the biodegradation or erosion of the biodegradable polymer, the drug diffuses out from the implant, achieving the release of the drug. Therefore, when the porosity and the average pore size of the buprenorphine implant are controlled within the specified ranges, the buprenorphine implant releases the drug in a sustained and stable manner, thereby reducing the occurrence of drug burst release and delayed release from the implant.

### Brief Description of the Drawings

To better describe and illustrate the embodiments or examples provided in the present application, reference may be made to one or more drawings. The additional details or examples used to describe the drawings should not be considered as limiting the scope of the disclosed application, the embodiments or examples currently described, or any of the optimal modes understood for these applications. Furthermore, the same numeral signs are used to denote the same components throughout the drawings. In the drawings:
FIG. 1 is a schematic scanning electron microscopy (SEM) image of a cross section of a buprenorphine implant prepared in Example 1;
FIG. 2 is a schematic SEM image of a cross section of a buprenorphine implant prepared in Example 2;
FIG. 3 is a schematic SEM image of a cross section of a buprenorphine implant prepared in Example 3;
FIG. 4 is a schematic SEM image of a cross section of a buprenorphine implant prepared in Comparative Example 1;
FIG. 5 is a schematic SEM image of a cross section of a buprenorphine implant prepared in Comparative Example 2;
FIG. 6 is a schematic SEM image of a cross section of a buprenorphine implant prepared in Comparative Example 5;
FIG. 7 is a schematic in-vitro release curve chart of buprenorphine implants prepared in Examples 1, 2, 4, 6, and 8 and Comparative Examples 1, 2, 5, and 6; and
FIG. 8 is a schematic in-vivo release curve chart of buprenorphine implants prepared in Example 6 and Comparative Example 5 in rats.

### Detailed Description of the Embodiments

In order to facilitate an understanding of the present application, a more complete description of the present application will be made below. Preferred embodiments are provided in the accompanying drawings. However, the present application may be implemented in many different forms and should not be limited to the embodiments herein. Rather, these embodiments are provided for the purpose of providing a more thorough and comprehensive understanding of the disclosure of the present application.

Unless otherwise defined, all technical and scientific terms used herein have the same meaning as commonly understood by those skilled in the art to which the present application belongs. Terms used in the description of the present application are for the purpose of describing specific embodiments only and are not intended to limit the present application.

In the present application, the technical features described in an open-ended manner include not only closed technical solutions composed of the listed features but also open-ended technical solutions that encompass the listed features.

In the present application, when numerical ranges are mentioned, unless specifically stated otherwise, such ranges are considered continuous and include both minimum and maximum values of the ranges, as well as every value between the minimum and maximum values. Further, when the range refers to integers, it includes every integer between the minimum and maximum values. Additionally, when a plurality of ranges are provided to describe features or characteristics, these ranges may be combined. In other words, unless otherwise specified, all ranges disclosed herein should be understood to include any and all subranges encompassed within them.

The present application specifically discloses some numerical ranges. However, any lower limit may be combined with any upper limit to form an undisclosed range, and any lower limit may be combined with any other lower limit to form an undisclosed range, similarly, any upper limit may be combined with any other upper limit to form an undisclosed range. Moreover, each individually disclosed point or value may serve as a lower or upper limit in combination with any other point or value or in combination with any other lower or upper limit to form an undisclosed range.

In the present application, temperature parameters, unless specifically limited, allow for either constant temperature treatment or treatment within a certain temperature range. The constant temperature treatment permits temperature fluctuations within the control accuracy range of the instrument.

In the descriptions of the application, "a plurality of' means at least two, for example, two and three, unless otherwise limited definitely and specifically.

Unless specifically stated, all implementations and optional implementations of the present application may be combined to form a new technical solution. Unless specifically stated, all technical features and optional technical features of the present application may be combined to form a new technical solution.

Unless specifically stated, all steps in the present application may be performed sequentially or in a random order, and are preferably performed sequentially.

One or more embodiments of the present application provide a buprenorphine implant, which includes: a biodegradable polymer and an active pharmaceutical ingredient dispersed in the biodegradable polymer. The active pharmaceutical ingredient includes one or more of buprenorphine and a pharmaceutically acceptable salt thereof.

A plurality of pores are formed in an interior of the buprenorphine implant, with a porosity of 2% to 10% and an average pore size of 2 µm to 15 µm.

It should be noted that the active pharmaceutical ingredient is dispersed in the biodegradable polymer in the form of a plurality of crystalline particles.

The interior of the buprenorphine implant refers to all parts of the buprenorphine implant except for an outer surface.

The active pharmaceutical ingredient is partially dispersed in the biodegradable polymer and partially distributed on the surface of the biodegradable polymer. The abovementioned porosity of the buprenorphine implant ranges from 2% to 10%; and for example, the porosity of the buprenorphine implant may be, but is not limited to, 2%, 2.5%, 3%, 3.5%, 4%, 4.5%, 5%, 5.5%, 6%, 6.5%, 7%, 7.5%, 8%, 8.5%, 9%, 9.5%, or 10%, or within any range between any two of these values.

The abovementioned average pore size of the buprenorphine implant ranges from 2 µm to 15 µm, and may be, but is not limited to, 2 µm, 2.5 µm, 3 µm, 3.5 µm, 4 µm, 4.5 µm, 5 µm, 5.5 µm, 6 µm, 6.5 µm, 7 µm, 7.5 µm, 8 µm, 8.5 µm, 9 µm, 9.5 µm, 10 µm, 11 µm, 11.5 µm, 12 µm, 12.5 µm, 13 µm, 13.5 µm, 14 µm, 14.5 µm, or 15 µm, or within any range between any two of these values.

It may be understood that when the buprenorphine implant is in a vivo, the biodegradation or erosion of the biodegradable polymer is facilitated by a subcutaneous tissue fluid or enzyme-catalyzed reactions. During the biodegradation or erosion of the biodegradable polymer, the drug diffuses out from the implant, achieving the release of the drug. It is also readily understood that buprenorphine on the outer surface of the implant is released first, constituting an initial phase of drug release. Subsequently, with the biodegradation or erosion of the biodegradable polymer, buprenorphine is gradually released from the interior of the implant, forming middle and later phases of drug release. When the porosity and the average pore size of the buprenorphine implant fall within the abovementioned ranges, a suitable number of pathways are provided for the biodegradation or erosion of the biodegradable polymer by a subcutaneous tissue fluid or enzyme-catalyzed reactions, that is, a suitable number of channels are provided for drug diffusion from the interior of the implant. Particularly in the later phase of drug release, a tissue fluid may infiltrate the implant through the pores and the enzymes in the tissue fluid catalyze the biodegradation or erosion of the biodegradable polymer in a region farther away from the surface of the implant. This allows the drug in the implant to continue releasing in a sustained and stable manner, thereby reducing or even preventing both burst release and delayed release throughout the drug release process of the implant.

Furthermore, the formulation is simple and does not involve the use of organic solvents, and need for surgical implant removal is eliminated, thereby improving patient compliance.

In some embodiments, the active pharmaceutical ingredient accounts for 30% to 70% of the total mass of the active pharmaceutical ingredient and the biodegradable polymer. For example, it may be, but is not limited to, 30%, 35%, 40%, 45%, 50%, 55%, 60%, 65%, or 70%, or within any range between any two of these values. The active pharmaceutical ingredient is dispersed in the biodegradable polymer in the form of a plurality of crystalline particles, with each crystalline particle being encapsulated by the biodegradable polymer. The distribution of the active pharmaceutical ingredient in the biodegradable polymer is influenced by the content of the active pharmaceutical ingredient, and affects the degree to which the crystalline particles are encapsulated by the biodegradable polymer. The degree of encapsulation of the crystalline particles by the biodegradable polymer affects a quantity and size of pores, that is, the dispersion of the crystalline particles in the biodegradable polymer influences the porosity and pore size. When the mass percentage of the active pharmaceutical ingredient in the total mass of the active pharmaceutical ingredient and the biodegradable polymer falls within the abovementioned range, the formation of a buprenorphine implant with porosity and pore size within the desired ranges is facilitated, which, in turn, contributes to the stable and sustained release of the active pharmaceutical ingredient from the implant.

When the mass percentage of the active pharmaceutical ingredient in the total mass of the active pharmaceutical ingredient and the biodegradable polymer exceeds the abovementioned range, an excessively high porosity of the implant and an increased drug release rate may occur, thereby leading to a higher risk of burst release in the initial phase. When the mass percentage of the active pharmaceutical ingredient in the total mass of the active pharmaceutical ingredient and the biodegradable polymer is below the abovementioned range, an excessively low porosity of the implant and a reduced drug release rate may occur, thereby leading to a higher risk of delayed release.

Optionally, the active pharmaceutical ingredient accounts for 35% to 65% of the total mass of the active pharmaceutical ingredient and the biodegradable polymer, and further optionally, the active pharmaceutical ingredient accounts for 40% to 60% of the total mass of the active pharmaceutical ingredient and the biodegradable polymer.

As a possible implementation, a particle size of the active pharmaceutical ingredient is ≤30 mesh. The particle size of the active pharmaceutical ingredient affects the porosity and pore size of the prepared buprenorphine implant. When the particle size of the active pharmaceutical ingredient is ≤30 mesh, the porosity and the average pore size of the prepared buprenorphine implant can be maintained within the abovementioned ranges respectively. When the particle size exceeds this range, the porosity of the buprenorphine implant may significantly increase, thereby leading to larger pores and an elevated risk of drug burst release from the implant. Additionally, when the particle size of the active pharmaceutical ingredient is greater than 30 mesh, the drug release rate of the implant may increase, thereby shortening an administration cycle and potentially making it difficult to achieve a desired sustained-release effect.

Optionally, the particle size of the active pharmaceutical ingredient is ≤40 mesh; and further optionally, the particle size of the active pharmaceutical ingredient is ≤50 mesh.

In some possible implementations, the biodegradable polymers used in this application, such as polylactic acid, poly(lactide-co-glycolide), polycaprolactone, and polyethylene glycol block copolymers thereof, are all in the amorphous form with a typical glass transition temperature of 35°C to 65°C. For example, the glass transition temperature may be, but is not limited to, 35°C, 40°C, 45°C, 50°C, 55°C, 60°C, or 65°C, or within any range between two of these values. Buprenorphine and the pharmaceutically acceptable salt thereof are crystalline substances with a melting point above 260°C. When the glass transition temperature of the biodegradable polymer falls within the abovementioned range, during the hot-melt extrusion (where the hot-melt temperature is significantly lower than the melting point of buprenorphine) of the mixture of the active pharmaceutical ingredient and the biodegradable polymer, crystalline buprenorphine is uniformly dispersed in the molten polymer, forming a crystalline solid dispersion. Therefore, effective dispersion of the active pharmaceutical ingredient crystalline particles in the biodegradable polymer is achieved.

Further, since the glass transition temperature of the biodegradable polymer falls within the abovementioned range, the extrusion of the biodegradable polymer and the active pharmaceutical ingredient can be performed without requiring high temperatures or solvents. This minimizes thermal degradation and hydrolysis of the active pharmaceutical ingredient or the biodegradable polymer, thereby preparing the buprenorphine implant with low impurity level, uniform content, and good stability during storage.

In some possible implementations, an end-capping group of the biodegradable polymer is an alkyl ester group or a carboxyl group. The end-capping group directly influences the polarity of the biodegradable polymer, and a polarity relationship between the biodegradable polymer and the active pharmaceutical ingredient affects a porosity and pore size of a solid solution formed during the hot-melt extrusion. According to the principle of "like dissolves like", when the polarities of the biodegradable polymer and the active pharmaceutical ingredient are similar, good compatibility of the biodegradable polymer and the active pharmaceutical ingredient results in a lower porosity and smaller average pore size of the solid solution formed during hot-melt extrusion. When the end-capping group of the biodegradable polymer is the alkyl ester group or the carboxyl group, the porosity and pore size of the prepared buprenorphine implant are maintained in the specified ranges respectively, thereby facilitating the stable release of the active pharmaceutical ingredient from the implant.

In some implementations, a weight-average molecular weight of the biodegradable polymer is 5,000 to 100,000 Da. For example, the weight-average molecular weight of the biodegradable polymer may be, but is not limited to, 5,000 Da, 10,000 Da, 20,000 Da, 30,000 Da, 40,000 Da, 50,000 Da, 60,000 Da, 70,000 Da, 80,000 Da, 90,000 Da, or 100,000 Da, or within any range between any two of these values. Generally, the weight-average molecular weight of the biodegradable polymer influences a drug release duration of the implant. A higher weight-average molecular weight of the biodegradable polymer results in a longer drug release duration of the implant, while a lower weight-average molecular weight of the biodegradable polymer results in a shorter drug release duration of the implant.

In some possible implementations, an intrinsic viscosity of the biodegradable polymer ranges from 0.08 to 0.6 dl/g. For example, the intrinsic viscosity of the biodegradable polymer may be, but is not limited to, 0.08 dl/g, 0.1 dl/g, 0.2 dl/g, 0.3 dl/g, 0.4 dl/g, 0.5 dl/g, or 0.6 dl/g, or within any range between any two of these values. The intrinsic viscosity of the biodegradable polymer affects the drug release duration of the implant, where a higher intrinsic viscosity of the biodegradable polymer results in a longer drug release duration of the implant, while a lower intrinsic viscosity of the biodegradable polymer results in a shorter drug release duration of the implant.

As one possible implementation, the biodegradable polymer includes one or more of polylactide, poly(lactide-co-glycolide), polylactide and polyethylene glycol copolymers, and poly(lactide-co-glycolide) and polyethylene glycol copolymers. Optionally, the biodegradable polymer includes one or more of polylactide and poly(lactide-co-glycolide). Further optionally, the biodegradable polymer includes poly(lactide-co-glycolide).

In some implementations, the poly(lactide-co-glycolide) includes a lactide unit and a glycolide unit, and the molar percentage of the lactide unit in the total molar amount of the lactide unit and the glycolide unit is denoted as R, where 50% ≤ R < 100%. For example, R may be, but is not limited to, 50%, 55%, 60%, 65%, 70%, 75%, 80%, 85%, 90%, 95%, 99%, or 99.99%, or within any range between any two of these values. When the molar percentage of the lactide unit in the total molar amount of the lactide unit and the glycolide unit falls within this range, the porosity and average pore size of the prepared buprenorphine implant are within the specified ranges. Optionally, R may be in the range of 65% ≤ R < 100%.

In some implementations, the pharmaceutically acceptable salt of buprenorphine includes one or more of buprenorphine hydrochloride, buprenorphine salicylate, buprenorphine sulfate, buprenorphine fumarate, buprenorphine oxalate, buprenorphine hydrobromide, buprenorphine tartrate, buprenorphine maleate, and buprenorphine phosphate; and optionally, the pharmaceutically acceptable salt of buprenorphine may include buprenorphine hydrochloride.

In some implementations, the buprenorphine implant is cylindrical or rod-shaped; and optionally, the buprenorphine implant has a diameter of ≤ 3 mm.

It should be noted that a length of the buprenorphine implant may be determined based on the desired drug release duration. A longer buprenorphine implant can extend the drug release duration, whereas a shorter buprenorphine implant can shorten the drug release duration.

One or more embodiments of the present application further provide a preparation method of the buprenorphine implant, which includes the following steps: a mixture of an active pharmaceutical ingredient and a biodegradable polymer is prepared; and hot-melt extrusion is performed on the mixture to prepare the buprenorphine implant.

In some implementations, the step that a mixture of an active pharmaceutical ingredient and a biodegradable polymer is prepared includes: the active pharmaceutical ingredient and the biodegradable polymer are cryogenically milled, dried, sieved, and mixed to prepare the mixture.

In some implementations, a particle size of the active pharmaceutical ingredient and a particle size of the biodegradable polymer in the mixture are ≤30 mesh. When the particle size of the active pharmaceutical ingredient is more than 30 mesh, the porosity of the prepared buprenorphine implant may be higher than 10%, which may increase a risk of drug burst release from the implant. Additionally, the drug release rate may also be increased, which leads to a shortened administration duration and may make it difficult to achieve the desired sustained-release effect.

Optionally, the particle size of the active pharmaceutical ingredient and the particle size of the biodegradable polymer in the mixture are ≤40 mesh. Further optionally, the particle size of the active pharmaceutical ingredient and the particle size of the biodegradable polymer in the mixture are ≤50 mesh.

In some embodiments, moisture content of the active pharmaceutical ingredient and moisture content of the biodegradable polymer in the mixture are independently 1.5 wt% to 4.5 wt%. For example, it may be, but is not limited to, 1.5 wt%, 1.8 wt%, 2 wt%, 2.3 wt%, 2.5 wt%, 2.7 wt%, 3 wt%, 3.3 wt%, 3.5 wt%, 3.8 wt%, 4 wt%, 4.3 wt%, or 4.5 wt%, or within any range between any two of these values. The presence of water in the active pharmaceutical ingredient and the biodegradable polymer in the mixture allows for the formation of pores in the buprenorphine implant after water evaporation during hot-melt extrusion. These pores can serve as pathways for the biodegradation or erosion of the biodegradable polymer by a subcutaneous tissue fluid or enzyme-catalyzed reactions, thereby providing channels for drug diffusion and enabling sustained drug release in the middle and later phases. When the moisture content of the active pharmaceutical ingredient and the moisture content of the biodegradable polymer in the mixture fall within the above range, the porosity of the prepared buprenorphine implant is maintained between 2% and 10%.

In some implementations, during hot-melt extrusion, a melt temperature is 95°C to 120°C. For example, the melt temperature may be, but is not limited to, 95°C, 100°C, 105°C, 110°C, 115°C, or 120°C, or within any range between any two of these values. Since high temperature is not required for hot-melt extrusion, thermal degradation of the active pharmaceutical ingredient or the biodegradable polymer may be minimized, thereby preparing the implant with low impurity level, uniform content, and stable quality during storage.

In some embodiments, during hot-melt extrusion, a twin-screw torque is 35% to 80%. For example, it may be, but is not limited to, 35%, 40%, 45%, 50%, 55%, 60%, 65%, 70%, 75%, or 80%, or within any range between any two of these values.

In some embodiments, during hot-melt extrusion, a die pressure is 30 Bar to 70 Bar. For example, it may be, but is not limited to, 30 Bar, 35 Bar, 40 Bar, 45 Bar, 50 Bar, 55 Bar, 60 Bar, 65 Bar, or 70 Bar, or within any range between any two of these values.

The twin-screw torque and the die pressure are influenced by factors such as melt temperature, drug loading, and particle size. For example, when the melt temperature increases, the twin-screw torque and the die pressure may correspondingly decrease, which in turn may lead to a reduction in the porosity of the prepared buprenorphine implant. Similarly, when the mass percentage of the active pharmaceutical ingredient in the total mass of the active pharmaceutical ingredient and the biodegradable polymer decreases, the twin-screw torque and the die pressure may also decrease, resulting in a lower porosity of the buprenorphine implant. When the melt temperature, the twin-screw torque, the die pressure, and the particle size fall within the abovementioned ranges, the porosity of the prepared buprenorphine implant is maintained between 2% and 10%.

In some implementations, a parameter setting during hot-melt extrusion includes a twin-screw rotation speed of 50 rpm to 300 rpm. For example, the twin-screw rotation speed may be, but is not limited to, 50 rpm, 70 rpm, 100 rpm, 130 rpm, 150 rpm, 170 rpm, 200 rpm, 230 rpm, 250 rpm, 280 rpm, or 300 rpm, or within any range between any two of these values. The twin-screw rotation speed affects the intensity of shear mixing force applied to the material, which in turn influences the generated mechanical energy. The magnitude of the mechanical energy can affect the particle size of the active pharmaceutical ingredient dispersed in the biodegradable polymer, thereby impacting the porosity of the prepared buprenorphine implant. When the twin-screw rotation speed increases, the shear mixing force applied to the material may become more intense, thereby generating high mechanical energy. Thus, the active pharmaceutical ingredient is dispersed in the biodegradable polymer in smaller particle sizes or even molecular form, thereby reducing the porosity of the prepared buprenorphine implant and prolonging the drug release duration. When the twin-screw rotation speed falls within the abovementioned range, the porosity, pore size, and pore distribution uniformity of the buprenorphine implant are maintained in the desired ranges. Optionally, the twin-screw rotation speed is 80 rpm to 250 rpm.

In some implementations, after hot-melt extrusion is performed on the mixture, cooling, shaping, and pelletizing are performed to prepare a cylindrical buprenorphine implant.

The provided preparation method of the buprenorphine implant involves fewer process steps, eliminates the need for complex process devices, and is more conducive to achieving sterile production. By synergistically controlling process parameters such as the particle size of the active pharmaceutical ingredient and the particle size of the biodegradable polymer, the twin-screw rotation speed, the melt temperature, the twin-screw torque, and the die pressure, the porosity and average pore size of the prepared buprenorphine implant can be maintained in the defined ranges. This ensures stable drug release of the implant while reducing the occurrence of burst release or delayed release.

The technical solutions of the present application will be described in details with reference to specified embodiments.

### I. Preparation of buprenorphine implant

### Example 1

18 g of buprenorphine hydrochloride (serving as an active pharmaceutical ingredient) and 42.0 g of polylactic acid (serving as a biodegradable polymer with a weight-average molecular weight of 25 kDa and an intrinsic viscosity of 0.22 dL/g,) were cryogenically milled at -30°C, with moisture content controlled at 2%. The milled material was sieved, and particles of ≤40 mesh were collected to prepare a mixture. The active pharmaceutical ingredient accounted for 30% of the total mass of the active pharmaceutical ingredient and the biodegradable polymer, while the biodegradable polymer accounted for 70% of the total mass of the active pharmaceutical ingredient and the biodegradable polymer.

The mixture was then subjected to hot-melt extrusion by using a Thermo Fisher Pharma 11 co-rotating twin-screw hot-melt extruder. The screw speed was set to 150 rpm, the melt temperature during the hot-melt extrusion was 108°C, the twin-screw torque was maintained at 50% to 60%, and the die pressure ranged from 40 Bar to 45 Bar.

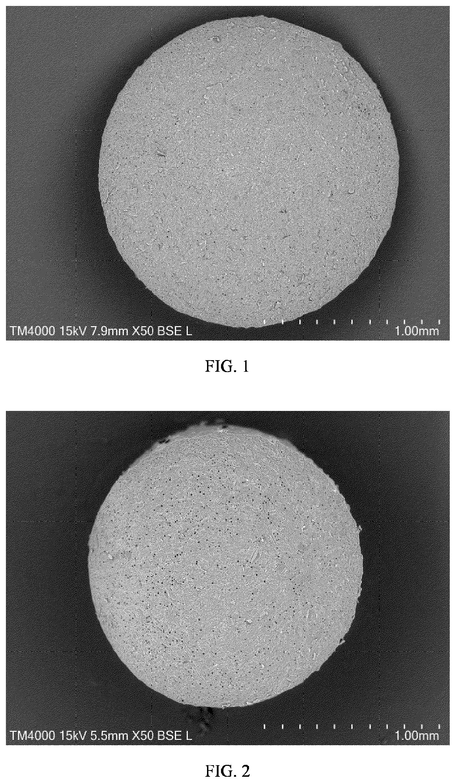

The extruded material was pelletized to obtain a cylindrical implant with a diameter of 1.5 mm. Scanning electron microscopy (SEM) revealed internal pore distribution of the implant, as shown in FIG. 1. Mercury intrusion porosimetry determined that the implant had a porosity of 2.4% and an average pore size of 3.5 µm.

### Examples 2 to 13 and Comparative Examples 1 to 9

The preparation method of the buprenorphine implant in Examples 2 to 13 and Comparative Examples 1 to 9 is different from that in Example 1 in at least one of the following aspects: the type and/or the mass percentage in the total mass of the active pharmaceutical ingredient and the biodegradable polymer and/or the particle size of the active pharmaceutical ingredient; the type and/or the mass percentage in the total mass of the active pharmaceutical ingredient and the biodegradable polymer and/or the particle size of the biodegradable polymer; the moisture content of the active pharmaceutical ingredient and the moisture content of the biodegradable polymer during preparation; parameter settings of the melt temperature and/or the twin-screw torque and/or the die pressure and/or twin-screw speed during hot-melt extrusion; and the porosity and/or the average pore size of the prepared implant. Specific details are shown in Table 1-1 and Table 1-2.

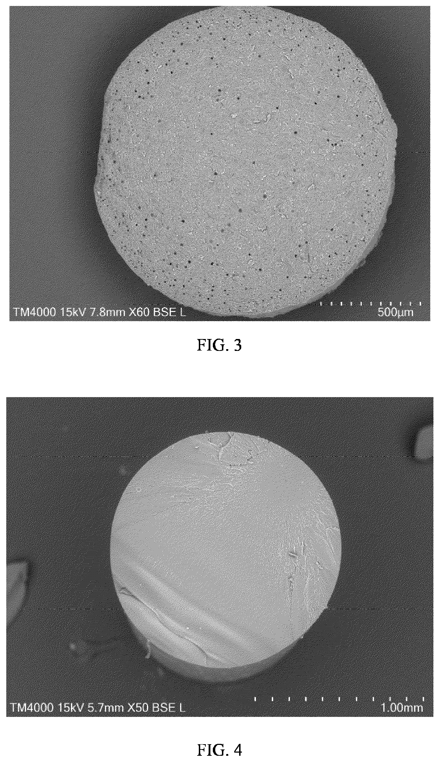

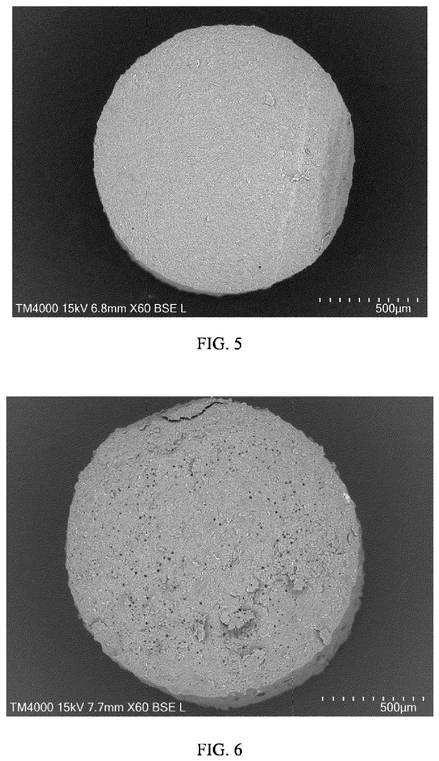

SEM was performed on the buprenorphine implants prepared in Examples 1 to 13 and Comparative Examples 1 to 9 to observe the internal pore distribution. An SEM image of a cross section of the buprenorphine implant prepared in Example 1 is shown in FIG. 1. An SEM image of a cross section of the buprenorphine implant prepared in Example 2 is shown in FIG. 2. An SEM image of a cross section of the buprenorphine implant prepared in Example 3 is shown in FIG. 3. An SEM image of a cross section of the buprenorphine implant prepared in Comparative Example 1 is shown in FIG. 4. An SEM image of a cross section of the buprenorphine implant prepared in Comparative Example 2 is shown in FIG. 5. An SEM image of a cross section of the buprenorphine implant prepared in Comparative Example 5 is shown in FIG. 6.

FIG. 1 to FIG. 3 demonstrate that a certain number of pores are uniformly distributed in the buprenorphine implants prepared in Examples 1 to 3. The buprenorphine implants prepared in Comparative Examples 1 and 2 contain only a small number of pores, while the buprenorphine implant in Comparative Example 5 exhibits an excessive number of pores.

**Table 1-1**

| Example | Preparation process | | | | | | | | | | | | Product | | | | |
|---|---|---|---|---|---|---|---|---|---|---|---|---|---|---|---|---|---|
| | API type | API particle size | Excipient type | Excipient particle size | Excipient weight-average molecular weight (kDa) | Excipient intrinsic viscosity (dL/g) | API moisture content | Excipient moisture content | Twin-screw rotatio n speed (rpm) | Melt temperat ure (°C) | Twin-screw torque | Die pressure (Bar) | w1 | w2 | Porosity | avera ge pore size (µm) | Diamet cr (mm) |
| Example 1 | Buprenorphine hydrochloride | ≤40 mesh | PLA | ≤40 mesh | 25 | 0.22 | 2 wt% | 2 wt% | 150 | 108 | 50-60% | 40-45 | 30% | 70% | 2.40% | 3.5 | 1.5 |
| Example 2 | Buprenorphine hydrochloride | ≤40 mesh | PLA | ≤40 mesh | 25 | 0.22 | 2 wt% | 2 wt% | 150 | 108 | 70-80% | 60-70 | 70% | 30% | 8.90% | 14.6 | 1.5 |
| Example 3 | Buprenorphine hydrochloride | ≤50 mesh | PLGA with a lactide-to-glycolide molar ratio of 75: 25, carboxyl-terminated | ≤50 mesh | 30 | 0.28 | 2 wt% | 2 wt% | 150 | 119 | 40-45% | 40-45 | 45% | 55% | 4.50% | 5.4 | 1.5 |
| Example 4 | Buprenorphine hydrochloride | ≤50 mesh | PLGA with a lactide-to-glycolide molar ratio of 75: 25, carboxyl-terminated | ≤50 mesh | 30 | 0.28 | 2 wt% | 2 wt% | 150 | 108 | 60-65% | 65-70 | 45% | 55% | 7.60% | 8.7 | 1.5 |
| Example 5 | Buprenorphine hydrochloride | ≤60 mesh | PLGA with a lactide-to-glycolide molar ratio of 65: 35, alkyl ester-terminated | ≤60 mesh | 30 | 0.26 | 2.5 wt% | 2.5 wt% | 150 | 110 | 60-65% | 65-70 | 55% | 45% | 5.50% | 9.5 | 1.5 |
| Example 6 | Buprenorphine hydrochloride | ≤60 mesh | PLGA with a lactide-to-glycolide molar ratio of 65: 35, alkyl ester-terminated | ≤60 mesh | 30 | 0.26 | 4.5 wt% | 4.5 wt% | 150 | 110 | 20-30% | 30-35 | 55% | 45% | 9.50% | 13.2 | 1.5 |
| Example 7 | Buprenorphine hydrochloride | ≤60 mesh | PLGA with a lactide-to-glycolide molar ratio of 50: 50, carboxyl-terminated | ≤60 mesh | 30 | 0.32 | 1.5 wt% | 1.5 wt% | 200 | 101 | 40-45% | 35-40 | 40% | 60% | 3.40% | 3.2 | 1.5 |
| Example 8 | Buprenorphine hydrochloride | ≤60 mesh | PLGA with a lactide-to-glycolide molar ratio of 50: 50, carboxyl-terminated | ≤60 mesh | 30 | 0.32 | 1.5 wt% | 1.5 wt% | 300 | 101 | 55-60% | 50-56 | 60% | 40% | 2.30% | 2.6 | 1.5 |
| Example 9 | Buprenorphine hydrochloride | ≤40 mesh | PLA | ≤40 mesh | 25 | 0.22 | 2 wt% | 2 wt% | 150 | 108 | 55-65% | 45-50 | 35% | 65% | 3.3 | 4.2 | 1.5 |
| Example 10 | Buprenorphine hydrochloride | ≤40 mesh | PLA | ≤40 mesh | 25 | 0.22 | 2 wt% | 2 wt% | 150 | 108 | 60-70% | 50-55 | 40% | 60% | 5.3 | 11.8 | 1.5 |

**Table 1-2**

| Example | Preparation process | | | | | | | | | | | | Product | | | | |
|---|---|---|---|---|---|---|---|---|---|---|---|---|---|---|---|---|---|
| | API type | API particl e size | Excipient type | Excipient particle size | Excipient weight-average molecular weight (kDa) | Excipient intrinsic viscosity (dL/g) | API moisture content | Excipient moisture content | Twin-screw rotation speed (rpm) | Melt temper ature (°C) | Twin-screw torque | Die pressure (Bar) | w1 | w2 | Porosity | avera ge pore size (µm) | Diamet er (mm) |
| Example 11 | Buprenorphine hydrochloride | ≤30 mesh | PLA | ≤30 mesh | 25 | 0.22 | 2 wt% | 2 wt% | 150 | 108 | 55-65% | 40-48 | 30% | 70% | 5.9 | 13.2 | 1.5 |
| Example 12 | Buprenorphine hydrochloride | ≤50 mesh | PLA | ≤50 mesh | 25 | 0.22 | 2 wt% | 2 wt% | 150 | 108 | 45-55% | 38-42 | 30% | 70% | 2.2 | 3.1 | 1.5 |
| Cpmparat ive Example 1 | Buprenorphine hydrochloride | ≤40 mesh | PLA | ≤40 mesh | 25 | 0.22 | 2 wt% | 2 wt% | 150 | 103 | 20-30% | 20-25 | 20% | 80% | 0.50% | 0.8 | 1.5 |
| Cpmparat ive Example 2 | Buprenorphine hydrochloride | ≤50 mesh | PLGA with a lactide-to-glycolide molar ratio of 75:25, carboxyl-terminated | ≤50 mesh | 30 | 0.28 | 2 wt% | 2 wt% | 150 | 124 | 15-25% | 15-30 | 45% | 55% | 1.10% | 1.2 | 1.5 |
| Cpmparat ive Example 3 | Buprenorphine hydrochloride | ≤60 mesh | PLGA with a lactide-to-glycolide molar ratio of 65:35, alkyl ester- | ≤60 mesh | 30 | 0.26 | 1 wt% | 1 wt% | 150 | 110 | 50-55% | 55-60 | 55% | 45% | 1.80% | 1.6 | 1.5 |
| Cpmparat ive Example 4 | Buprenorphine hydrochloride | ≤60 mesh | PLGA with a lactide-to-glycolide molar ratio of 50:50, carboxyl-terminated | ≤60 mesh | 30 | 0.32 | 1.50% | 1.50% | 40 | 101 | 40-45% | 20-30 | 60% | 40% | 11.00% | 17.7 | 1.5 |
| Cpmparat ive Example 5 | Buprenorphine hydrochloride | ≤20 mesh | PLGA with a lactide-to-glycolide molar ratio of 65:35, alkyl ester-terminated | ≤20 mesh | 30 | 0.26 | 2.50% | 2.50% | 150 | 108 | 70-85% | 70-80 | 55% | 45% | 12.40% | 22.6 | 1.5 |
| Cpmparativ e Example 6 | Buprenorphine hydrochloride | ≤60 mesh | PLGA with a lactide-to-glycolide molar ratio of 45:55, carboxyl-terminated | ≤60 mesh | 30 | 0.32 | 1.50% | 1.50% | 300 | 101 | 30-35% | 20-30 | 60% | 40% | 1.30% | 1.7 | 1.5 |
| Cpmparativ e Example 7 | Buprenorphine hydrochloride | ≤40 mesh | PLA | ≤40 mesh | 25 | 0.22 | 2 wt% | 2 wt% | 150 | 108 | 15-25% | 10-15 | 20% | 80% | 0.60% | 0.6 | |
| Cpmparativ e Example 8 | Buprenorphine hydrochloride | <40 mesh | PLA | ≤40 mesh | 25 | 0.22 | 2 wt% | 2 wt% | 150 | 108 | 75-86% | 66-76 | 80% | 20% | 11.30% | 23.1 | |
| Cpmparativ e Example 9 | Buprenorphine hydrochloride | ≤20 mesh | PLA | ≤20 mesh | 25 | 0.22 | 2 wt% | 2 wt% | 150 | 108 | 60-70% | 50-60 | 30% | 70% | 10.50% | 16.4 | |

Where "API type" refers to the type of the active pharmaceutical ingredient; "API particle size" refers to the particle size of the active pharmaceutical ingredient; "API moisture content" refers to the moisture content of the active pharmaceutical ingredient; "w1" represents the mass percentage of the active pharmaceutical ingredient in the total mass of the active pharmaceutical ingredient and the biodegradable polymer in the buprenorphine implant. "Excipient type" refers to the type of the biodegradable polymer; "Excipient particle size" refers to the particle size of the biodegradable polymer; "Excipient moisture content" refers to the moisture content of the biodegradable polymer; "Excipient weight-average molecular weight" refers to the weight-average molecular weight of the biodegradable polymer; "Excipient intrinsic viscosity" refers to the intrinsic viscosity of the biodegradable polymer; and "w2" represents the mass percentage of the biodegradable polymer in the total mass of the active pharmaceutical ingredient and the biodegradable polymer in the buprenorphine implant. "PLA" denotes polylactic acid, and "PLGA" denotes poly(lactic-co-glycolide).

From results shown in Table 1, it can be seen that the buprenorphine implants prepared by the preparation method provided by the present application exhibit a suitable porosity and average pore size.

The primary differences among Example 1, Examples 9 and 10, and Comparative Examples 7 and 8 lie in the mass ratio of the active pharmaceutical ingredient to the biodegradable polymer. In Comparative Example 7, the active pharmaceutical ingredient has the lowest mass percentage in the total mass of the active pharmaceutical ingredient and the biodegradable polymer, whereas in Comparative Example 8, the active pharmaceutical ingredient has the highest mass percentage in the total mass of the active pharmaceutical ingredient and the biodegradable polymer. Results of Example 1, Examples 9 and 10, and Comparative Examples 7 and 8 demonstrate that as the mass percentage of the active pharmaceutical ingredient in the total mass of the active pharmaceutical ingredient and the biodegradable polymer increases, the porosity and average pore size of the prepared buprenorphine implant also increase. When the mass percentage of the active pharmaceutical ingredient in the total mass of the active pharmaceutical ingredient and the biodegradable polymer falls within the range of 30% to 70%, the buprenorphine implant exhibits a suitable porosity, along with a suitable average pore size and uniform pore distribution.

The primary difference among Example 1, Examples 11 and 12, and Comparative Example 9 lies in the particle size of the active pharmaceutical ingredient, and the particle size of the active pharmaceutical ingredient in Comparative Example 9 is the largest. Results of Example 1, Examples 11 and 12, and Comparative Example 9 demonstrate that as the particle size of the active pharmaceutical ingredient increases, the porosity and average pore size of the prepared buprenorphine implant also increase. When the particle size of the active pharmaceutical ingredient is ≤30 mesh, the buprenorphine implant exhibits a suitable porosity, along with a suitable average pore size.

The primary differences among Examples 3 and 4 and Comparative Example 2 lie in the melt temperature, the twin-screw torque, and the die pressure. A comparison of results of Examples 3 and 4 and Comparative Example 2 indicates that as the melt temperature increases, the twin-screw torque and the die pressure decrease accordingly, leading to a reduction in the porosity and average pore size of the implant. When the melt temperature exceeds 120°C, both the twin-screw torque and the die pressure decrease excessively, ultimately resulting in an implant porosity of less than 2%.

The primary difference among Examples 5 and 6 and Comparative Example 3 lies in the moisture content of the active pharmaceutical ingredient and the moisture content of the biodegradable polymer in the mixture. A comparison of results of Examples 5 and 6 and Comparative Example 3 indicates that as the moisture content of the active pharmaceutical ingredient and the moisture content of the biodegradable polymer increase, the porosity and average pore size of the prepared implant also increase. The inventors of the present application believe that this phenomenon may be attributed to the fact that with the evaporation of water from the active pharmaceutical ingredients and excipients during hot-melt extrusion, the pores are formed in the implant, and a higher moisture content of the active pharmaceutical ingredients and the excipients results in greater porosity and average pore size of the prepared implant.

The primary difference between Example 8 and Comparative Example 4 lies in the twin-screw speed during hot-melt extrusion. A comparison of results of Example 8 and Comparative Example 4 indicates that as the twin-screw speed increases, the porosity of the prepared implant decreases. The inventors of the present application believe that this phenomenon may be attributed to the fact that with the increase in the twin-screw speed, the shear mixing force applied to buprenorphine becomes more intense, resulting in a higher mechanical energy. Consequently, buprenorphine is dispersed in the biodegradable polymer in smaller particle sizes or even molecular form, leading to a reduction in both the porosity and the average pore size of the prepared implant.

The primary difference between Example 8 and Comparative Example 6 lies in the molar ratio of the lactide unit to the glycolide unit in PLGA. A comparison of the results of Example 8 and Comparative Example 6 indicates that as the molar ratio of the lactide unit to the glycolide unit decreases, both the porosity and the average pore size of the prepared implant also decrease.

### II. In-vitro release testing

It should be noted that to eliminate the influence of an implant length on drug release, the buprenorphine implants prepared in Example 1, Example 2, Example 4, Example 6, Example 8, Comparative Example 1, Comparative Example 2, Comparative Example 5, and Comparative Example 6 were standardized to the same length.

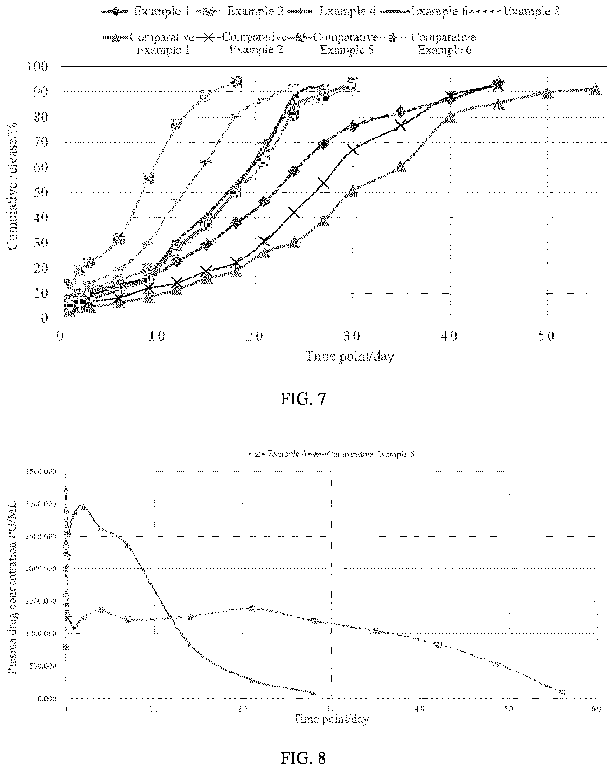

The in-vitro release testing was performed on the buprenorphine implants prepared in Example 1, Example 2, Example 4, Example 6, Example 8, Comparative Example 1, Comparative Example 2, Comparative Example 5, and Comparative Example 6. The specific procedure was as follows:
Six implants were respectively placed into 25 mL stoppered conical flasks, with 25 mL of water as a release medium, and the flasks were incubated in a thermostatic water bath shaker at 37°C ± 0.5°C, with an oscillation frequency of 50 rpm. The procedure was performed in accordance with established protocols. At predetermined time points (1, 2, 3, 6, 9, 12, 15, 18, 21, 24, 27, and 30 days), the entire release medium was removed and replaced with an equal volume of fresh release medium, ensuring that sink conditions were maintained throughout the release process. After 30 days, sampling intervals were set to 3 to 5 days and the sampling continued until the cumulative release amount reached 90% or more, at which sampling was stopped. The results of the in-vitro release testing are shown in Table 2 and FIG. 7.

**Table 2**

| Time point/day | Example 1 | Example 2 | Example 4 | Example 6 | Example 8 | Comparative Example 1 | Comparative Example 2 | Comparative Example 5 | Comparative Example 6 |
|---|---|---|---|---|---|---|---|---|---|
| 1 | 4.55 | 6.24 | 5.71 | 5.55 | 8.83 | 2.71 | 4.71 | 13.45 | 4.83 |
| 2 | 6.21 | 9.5 | 8.26 | 7.44 | 10.57 | 4.02 | 5.26 | 18.89 | 6.57 |
| 3 | 7.45 | 11.62 | 10.57 | 8.71 | 13.87 | 4.51 | 6.57 | 22.27 | 7.87 |
| 6 | 11.36 | 15.24 | 13.16 | 13.15 | 19.29 | 6.23 | 8.16 | 31.48 | 11.29 |
| 9 | 15.56 | 19.61 | 15.83 | 16.87 | 29.9 | 8.31 | 11.83 | 55.24 | 15.26 |
| 12 | 22.56 | 28.56 | 28.02 | 30.8 | 46.6 | 11.53 | 14.02 | 76.48 | 26.9 |
| 15 | 29.25 | 37.79 | 37.61 | 41.11 | 62.13 | 15.71 | 18.61 | 88.44 | 36.6 |
| 18 | 37.79 | 50.27 | 51.05 | 53.52 | 80.45 | 18.87 | 22.05 | 94.1 | 49.88 |
| 21 | 46.27 | 62.41 | 69.52 | 66.79 | 86.95 | 26.42 | 30.52 | / | 62.13 |
| 24 | 58.41 | 82.01 | 84.59 | 88.39 | 92.54 | 30.23 | 42.07 | | 80.45 |
| 27 | 69.35 | 89.06 | 89.07 | 92.61 | / | 38.79 | 53.52 | | 86.95 |
| 30 | 76.42 | 93.4 | 93.11 | / | | 50.56 | 66.79 | | 92.54 |
| 35 | 82.01 | / | / | | | 60.33 | 76.54 | | / |
| 40 | 87.06 | | | | | 80.16 | 88.39 | | |
| 45 | 93.43 | | | | | 85.52 | 92.61 | | |
| 50 | / | | | | | 89.73 | / | | |
| 55 | | | | | | 91.18 | | | |
| 60 | | | | | | / | | | |

A comparison of the results of Example 1 and 2 and Comparative Example 1 indicates that as the mass percentage of the active pharmaceutical ingredient increases, the porosity and average pore size of the buprenorphine implant increase accordingly, leading to a higher drug release rate and a shortened administration duration.

A comparison of the results of Example 6 and Comparative Example 5 indicates that when the particle size of the active pharmaceutical ingredient exceeds 30 mesh, the porosity of the buprenorphine implant increases significantly to more than 12.0%, and the average pore size also increases significantly to more than 22 µm. This results in more drug diffusion channels, an increased drug release rate, a higher risk of burst release, a shortened administration duration, and difficulty in achieving the long-acting formulation goal.

A comparison of the results of Example 4 and Comparative Example 2 indicates that when the melt temperature is above 120°C, the porosity of the buprenorphine implant is below 2%, and the average pore size is also below 2 µm. This leads to a significant reduction in the drug release rate, an increased risk of delayed release, and a plasma concentration in the initial phase of administration cannot achieve an effective therapeutic concentration.

A comparison of the results of Example 8 and Comparative Example 6 indicates that when the molar ratio of the lactide unit to the glycolide unit in PLGA is lower than 50: 50, the porosity of the buprenorphine implant is below 2%, and the average pore size is also below 2 µm. This results in a significant reduction in the drug release rate and an increased risk of delayed release.

### 3. Study on in-vivo drug release in rats

A method for determining buprenorphine concentration in biological samples from Sprague-Dawley (SD) rats by using LC-MS/MS was established. Two experimental groups were designed, namely, Example 6 and Comparative Example 5, with 6 rats in each group (3 males and 3 females). One buprenorphine implant (3.0 mg/implant) was subcutaneously implanted in the dorsal region. The drug administration frequency was a single dose for each group. Blood samples were collected at various time points after administration to measure the buprenorphine concentration, and the data were subjected to fitting analysis. The results are shown in Table 3 and FIG. 8.

**Table 3**

| Time point | | Example 6 | Comparative Example 5 |
|---|---|---|---|
| Concentration (pg/mL) | -1 h | BLLOQ | BLLOQ |
| | 5 min | 791.807 | 1468.247 |
| | 10 min | 1582.389 | 2409.409 |
| | 15 min | 2017.064 | 3220.006 |
| | 30 min | 2363.178 | 2928.945 |
| | 1 h | 2205.013 | 2898.986 |
| | 2 h | 2548.199 | 2787.360 |
| | 4 h | 2185.457 | 2669.673 |
| | 8 h | 1260.516 | 2565.629 |
| | 24 h | 1105.443 | 2869.385 |
| | 2 d | 1243.372 | 2955.136 |
| | 4 d | 1365.249 | 2619.598 |
| | 7 d | 1210.029 | 2359.126 |
| | 14 d | 1262.063 | 835.758 |
| | 21 d | 1388.209 | 278.519 |
| | 28 d | 1189.827 | 84.810 |
| | 35 d | 1039.732 | |
| | 42 d | 830.338 | |
| | 49 d | 512.089 | |
| | 56 d | 77.245 | |

The rat pharmacokinetic study reveals that under the condition where the implants have a same formulation (Example 6 and Comparative Example 5) but different internal porosity, the drug release profiles exhibit distinct behaviors after administration of the same dose. In Example 6, the buprenorphine implant has moderate porosity and average pore size. After a rapid onset, the plasma concentration stabilizes, a release profile approaches zero-order release kinetics, and stable sustained release for approximately two months can be ensured. In contrast, in Comparative Example 5, the buprenorphine implant has higher porosity and average pore size; after rapid release in the initial phase, due to the large number of pores in the implant, the subcutaneous tissue fluid infiltrates the implant through the pores, increasing the contact area with the polymer and accelerating its biodegradation rate; and as a result, the plasma concentration is higher, the release rate is faster, and the drug release duration is shorter.

In conclusion, by selecting the type and using amount of the active pharmaceutical ingredient and the biodegradable polymer, selecting the particle size of the active pharmaceutical ingredient, and optimizing key hot-melt extrusion parameters, the drug release rate of the buprenorphine implant can be controlled, thereby avoiding both burst release and delayed release and reducing peak-trough fluctuations. Formulations for different administration durations can be developed and requirements of different indications for various plasma concentration levels can be met. Furthermore, the entire formulation process avoids the use of organic solvents, thereby providing a safer and more effective hot-melt extrusion technology for implant production.

The technical features of the above embodiments may be freely combined. For brief description, not all possible combinations of the technical features in the above embodiments are described, but all the combinations of these technical features shall fall within the scope recorded in the description without conflicts.

The above embodiments only describe some implementations of the present application and are specifically described in details and not thus understood as limits to the patent scope of the present application. It is to be pointed out that those of ordinary skill in the art may further make a plurality of transformations and improvements without departing from the concept of the present application and all of these fall within the scope of protection of the present application. Therefore, the scope of patent protection of the present application should be subject to the appended claims.

## Claims

1. A buprenorphine implant, comprising:
a biodegradable polymer, and
an active pharmaceutical ingredient, dispersed in the biodegradable polymer and comprising one or more of buprenorphine and a pharmaceutically acceptable salt thereof, wherein
a plurality of pores are formed in an interior of the buprenorphine implant, with a porosity of 2% to 10% and an average pore size of 2 µm to 15 µm.

2. The buprenorphine implant according to claim 1, wherein the active pharmaceutical ingredient accounts for 30% to 70% of the total mass of the active pharmaceutical ingredient and the biodegradable polymer, optionally 35% to 65%, and further optionally 40% to 60%.

3. The buprenorphine implant according to claim 1 or 2, wherein a particle size of the active pharmaceutical ingredient is ≤30 mesh,
optionally, the particle size of the active pharmaceutical ingredient is ≤40 mesh, and
further optionally, the particle size of the active pharmaceutical ingredient is ≤50 mesh.

4. The buprenorphine implant according to any one of claims 1 to 3, wherein the biodegradable polymer has at least one of the following features:
(1) a glass transition temperature of the biodegradable polymer being 35°C to 65°C; and
(2) an end-capping group of the biodegradable polymer being an alkyl ester group or a carboxyl group.

5. The buprenorphine implant according to any one of claims 1 to 4, wherein the biodegradable polymer further has at least one of the following features:
(3) a weight-average molecular weight of the biodegradable polymer being 5,000 to 100,000 Da and optionally 15,000 to 70,000 Da; and
(4) an intrinsic viscosity of the biodegradable polymer being 0.08 to 0.6 dl/g and optionally 0.15 to 0.5 dl/g.

6. The buprenorphine implant according to any one of claims 1 to 5, wherein the biodegradable polymer comprises one or more of polylactide, poly(lactide-co-glycolide), polylactide and polyethylene glycol copolymers, and poly(lactide-co-glycolide) and polyethylene glycol copolymers;
optionally, the biodegradable polymer comprises one or more of polylactide and poly(lactide-co-glycolide); and
further optionally, the biodegradable polymer comprises poly(lactide-co-glycolide).

7. The buprenorphine implant according to claim 6, wherein poly(lactide-co-glycolide) comprises a lactide unit and a glycolide unit, and the molar percentage of the lactide unit in the total molar amount of the lactide unit and the glycolide unit is denoted as R, wherein 50% ≤ R < 100%, and optionally 65% ≤ R < 100%.

8. The buprenorphine implant according to any one of claims 1 to 7, wherein the pharmaceutically acceptable salt of buprenorphine comprises one or more of buprenorphine hydrochloride, buprenorphine salicylate, buprenorphine sulfate, buprenorphine fumarate, buprenorphine oxalate, buprenorphine hydrobromide, buprenorphine tartrate, buprenorphine maleate, and buprenorphine phosphate; and
optionally, the pharmaceutically acceptable salt of buprenorphine comprises buprenorphine hydrochloride.

9. The buprenorphine implant according to any one of claims 1 to 8, wherein the buprenorphine implant is cylindrical or rod-shaped; and
optionally, a diameter of the buprenorphine implant is ≤3 mm.

10. A preparation method of the buprenorphine implant according to any one of claims 1 to 9, comprising the following steps:
preparing a mixture of an active pharmaceutical ingredient and a biodegradable polymer; and
performing hot-melt extrusion on the mixture to prepare the buprenorphine implant.

11. The preparation method of the buprenorphine implant according to claim 10, wherein a particle size of the active pharmaceutical ingredient and a particle size of the biodegradable polymer in the mixture are independently ≤30 mesh;
optionally, the particle size of the active pharmaceutical ingredient and the particle size of the biodegradable polymer in the mixture are independently ≤40 mesh; and
further optionally, the particle size of the active pharmaceutical ingredient and the particle size of the biodegradable polymer in the mixture are independently ≤50 mesh.

12. The preparation method of the buprenorphine implant according to claim 10 or 11, wherein moisture content of the active pharmaceutical ingredient and moisture content of the biodegradable polymer in the mixture are independently 1.5 wt% to 4.5 wt%.

13. The preparation method of the buprenorphine implant according to any one of claims 10 to 12, wherein the hot-melt extrusion has at least one of the following features:
(1) a melt temperature of 95°C to 120°C;
(2) a twin-screw torque of 35% to 80%; and
(3) a die pressure of 30 Bar to 70 Bar.

14. The preparation method of the buprenorphine implant according to any one of claims 10 to 13, wherein a parameter setting during hot-melt extrusion comprises:
a twin-screw rotation speed of 50 rpm to 300 rpm; and optionally 80 rpm to 250 rpm.
